# EUROPÄISCHE PATENTSCHRIFT

(11) **EP 0 470 460 B1**
(45) Veröffentlichungstag und Bekanntmachung des Hinweises auf die Patenterteilung: **30.11.1994**
(21) Anmeldenummer: 91112633.2
(22) Anmeldetag: 27.07.1991
(51) Int. Cl.: C07C 251/20, C07C 249/02

(54) **Verfahren zur Herstellung von Azomethinen**
Process for the production of azomethines
Procédé de préparation d'azométhines

(30) Priorität: 09.08.1990 DE 4025185
(43) Veröffentlichungstag der Anmeldung: 12.02.1992
(73) Patentinhaber: BAYER AG, 51368 Leverkusen (DE)
(72) Erfinder: Langer, Reinhard, Dr., W-4150 Krefeld (DE); Buysch, Hans-Josef, Dr., W-4150 Krefeld (DE); Wagner, Paul, Dr., W-4000 Düsseldorf 13 (DE)

(56) Entgegenhaltungen:
- DE-A- 2 244 169
- DE-A- 2 244 238
- DE-A- 2 525 295
- DE-A- 2 901 863
- DE-C- 1 078 119

## Beschreibung

Die vorliegende Erfindung betrifft ein Verfahren zur Kondensation von gegebenenfalls substituierten Cycloalkanonen mit gegebenenfalls substituierten Anilinen unter Bildung von Azomethinen in kontinuierlicher Reaktion in einem kolonnenartigen Reaktor mit einem angelegten Temperaturprofil.

Die Kondensation von Cyclohexanon und Anilin zu Cyclohexylidenanilin mit Zinkchlorid als Katalysator ist seit langem bekannt (Ber. 53 (1920), 345-354). Für schwierige Fälle in solchen Kondensationen wurde das Katalysatorsystem HCl-ZnCl₂ eingesetzt (Ber. 46 (1913), 2718).

Die Kondensation von aromatischen Aminen mit Cyclohexanonen ist eine Gleichgewichtsreaktion mit geringer Wärmetönung. Sie wird zugunsten des Azomethins (Schiff'sche Base) verschoben, wenn man dem System das entstehende Reaktionswasser entzieht. Im allgemeinen erreicht man dies durch azeotrope Destillation, wozu gegegebenenfalls ein inertes Schleppmittel, wie Benzol, Toluol usw. eingesetzt wird. In jüngerer Zeit wurde zur Bindung des Reaktionswassers der Einsatz von TiCl₄ (J. Org. Chem. 32 (1967), 3247) oder von molaren Mengen (Butyl)₂SnCl₂ (Synth. Commun. 12 (1982), 495) beschrieben. Beide Verbindungen binden das entstehende Reaktionswasser unter Freisetzung von HCl.

Andere Entwicklungen gingen dahin, daß die das Wasser bindende Spezies kovalent an das Anilin gebunden ist, beispielsweise in Form von N,N-Bis-(trimethylsilyl)-anilin (Bull. Soc. Chim. Fr. 1966, 3205), Iminophosphoranen (Angew. Chem. Int. Ed. Eng. 5 (1966), 947) sowie N-(Diphenylaluminium)-anilin (J. Org. Chem. 51 (1986), 1848).

Eine weitere Methode, das entstehende Reaktionswasser wirkungsvoll zu binden und so bei geringen Anilin- oder Ketonüberschüssen unter milden Bedingungen arbeiten zu können, ist der Einsatz von Molekularsieben (J. Org. Chem. 36 (1971), 1570; DE-OS 2 244 238). Der Nachteil dieses zuletzt genannten Verfahrens ist die aufwendige und kostspielige Regenerierung des Molekularsiebs.

Die azeotrope Entwässerung ist somit sicherlich das technisch interessanteste Verfahren, wenn es gelingt, mit geringen Überachüssen an einer der beiden Komponenten zu akzeptablen Reaktionszeiten zu gelangen, ohne größere Mengen an Azeotrop-Schleppmittel einsetzen zu müssen.

In DE-AS 1 078 119 wird die Kondensation von N-Phenyl-p-phenylen-diamin mit Cyclohexanon ohne Katalysatorzusatz beschrieben; hierbei müssen Cyclohexanonüberschüsse von 200 bis 300 % eingesetzt werden.

In DE-OS 2 525 295 wird gezeigt, daß die Reaktionszeit der Kondensation von Anilin mit 400 % Überschuß Cyclohexanon ohne Katalysator mit steigender Ansatzgröße stark zunimmt, so daß eine Übersetzung in den technischen Maßstab dicht möglich ist. Ferner wurde gezeigt, daß stark- und schwachssaure organische Harze einen günstigen Einfluß auf die Reaktionszeit besitzen.

In DE-OS 2 901 863 werden frisch synthetisiertes, wasserfreies, nicht gebranntes Calciumhydrogenphosphat, Apatit der Formel Ca₅(PO₄)₃OH, getrocknete, nicht geglühte Aluminiumoxidhydroxide und protonengetauschte, neutral gewaschene Aluminiumsilikate vom Montmorillonit-Typ als wirkungsvolle Katalysatoren für die Umsetzung von aromatischen Aminen mit Ketonen beschrieben. Die Beispiele in dieser Patentanmeldung beschränken sich jedoch auf die Kondensation des reaktiven p-Phenylendiamins mit Methyl-isobutylketon, wobei ein Ketonüberschuß von 150 % angewandt wird.

Es war somit wünschenswert, ein preiswertes und umweltschonendes Verfahren zur Synthese von Cycloalkylidenanilinen zu entwickeln, welches sich durch hohe Ausbeuten, eine einfache Apparatur und optimale Energieausnutzung bei minimalem Energieeinsatz auszeichnet. Gefunden wurde ein Verfahren, mit dem bei minimalem Überschuß an Carbonyl- oder Anilinkomponente in einem kontinuierlichen Prozeß die Azomethinverbindung mit hoher Ausbeute und in hoher Reinheit gewonnen werden kann.

Es wurde ein Verfahren zur Herstellung von Azomethinen der Formel 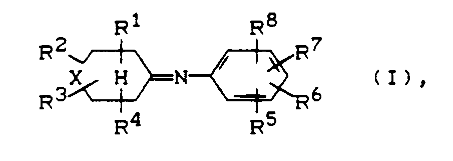
in der
- R¹, R², R³, R⁴, R⁵, R⁶, R⁷ und R⁸: unabhängig voneinander Wasserstoff, geradkettiges oder verzweigtes C₁-C₆-Alkyl, C₃-C₆-Cycloalkyl oder Aryl bedeuten und
- R⁵, R⁶, R⁷ und R⁸: unabhängig voneinander zusätzlich Halogen, geradkettiges oder verzweigtes C₁-C₆-alkoxy, Hydroxy, Amino, C₁-C₆-Alkylamino, Di-C₁-C₆-alkyl-amino, Aryloxy oder Arylamino bedeuten,
wobei Aryl für Phenyl oder für in 2-, 3- oder 4-Stellung gebundenes 5- oder 6-gliedriges Heteroaryl mit 1 oder 2 Heteroatomen aus der Gruppe N, O und S steht, und
X -CH₂- oder eine zwischen den benachbarten C-Atomen durchgehende Bindung bedeutet,
durch Kondensation von Cycloalkanonen der Formel 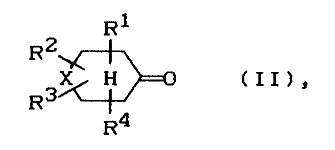
mit Anilinen der Formel 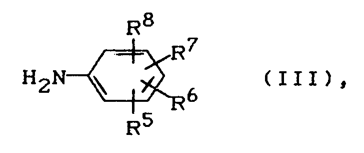
in denen R¹ bis R⁸ und X die obige Bedeutung haben,
in Gegenwart von sauren heterogenen Katalysatoren unter azeotroper Entfernung des Wassers gefunden, das dadurch gekennzeichnet ist, daß die Kondensation in kontinuierlicher Reaktion in einem kolonnenartigen Reaktor mit einem angelegten Temperaturprofil im Bereich von 10 bis 300°C durchgeführt wird, wobei die mit Katalysator gefüllten Reaktorabschnitte eine Temperatur von höchstens 250°C aufweisen und die Ausgangsstoffe im Niedertemperaturbereich zugeführt werden und von den Reaktionsprodukten das azeotrop zu entfernende Reaktionswasser ebenfalls im Niedertemperturbereich und das entstehende Azomethin im Hochtemperaturbereich abgeführt werden und wobei bei einem Molverhältnis von Cyclohexanon:Anilin = 2:1 bis 1:2 und in einem Druckbereich von 0,5 mbar bis 3 bar gearbeitet wird.

In bevorzugter Weise werden Ausgangsstoffe eingesetzt, in denen Phenyl für Aryl steht.

In weiterhin bevorzugter Weise werden Cycloalkanone eingesetzt, in denen R⁴ Wasserstoff bedeutet.

In noch weiterhin bevorzugter Weise werden Aniline eingesetzt, in denen R⁸ Wasserstoff bedeutet.

In besonders bevorzugter Form werden Cycloalkanone eingesetzt, in denen R³ und R⁴ Wasserstoff bedeuten.

In ebenfalls besonders bevorzugter Weise werden Aniline eingesetzt, in denen R⁷ und R⁸ Wasserstoff bedeuten.

Des weiteren sind Cycloalkanone der Formel 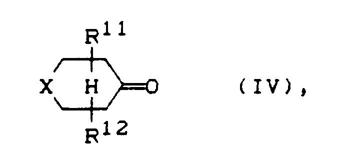
bevorzugt, in der
- R¹¹ und R¹²: unabhängig voneinander Wasserstoff, geradkettiges oder verzweigtes C₁-C₄-Alkyl, Cyclopropyl, Cyclopentyl, Cyclohexyl oder Phenyl bedeuten und
X -CH₂ oder eine zwischen den benachbarten C-Atomen durchgehende Bindung bedeutet.

In besonders bevorzugter Weise werden Cycloalkanone der Formel 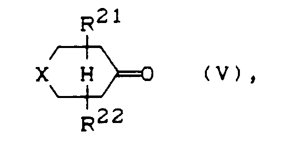
eingesetzt, worin
- R²¹ und R²²: unabhängig voneinander Wasserstoff, Methyl oder Ethyl bedeuten und
- X: die obige Bedeutung hat.

In bevorzugter Weise werden weiterhin Aniline der Formel 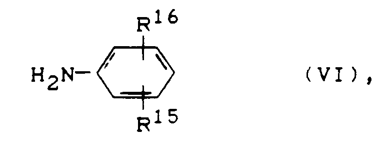
eingesetzt, in der
- R¹⁵ und R¹⁶: unabhängig voneinander Wasserstoff, geradkettiges oder verzweigtes C₁-C₄-Alkyl, Phenyl, Fluor, Chlor, Brom, geradkettiges oder verzweigtes C₁-C₄-Alkoxy, Hydroxy, Amino, C₁-C₄-Alkylamino, Di-C₁-C₄-alkyl-amino, Phenoxy oder Phenylamino bedeuten.

In besonders bevorzugter Weise werden Aniline der Formel 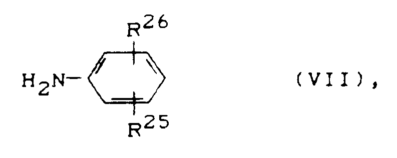
eingesetzt, in der
- R²⁵ und R²⁶: unabhängig voneinander Wasserstoff, Methyl, Ethyl, Fluor, Chlor, Methoxy, Ethoxy, Methylamino, Ethylamino, Dimethylamino oder Diethylamino bedeuten.

In ganz besonders bevorzugter Weise werden Aniline der Formel 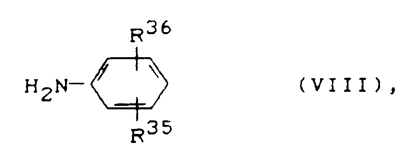
eingesetzt, in der
- R³⁵ und R³⁶: unabhängig voneinander Wasserstoff, Fluor, Chlor, Methyl, Ethyl, Methoxy, Methylamino oder Dimethylamino bedeuten.

Als Alkylgruppen in den genannten Substituenten (Alkyl, Alkoxy, Alkylamino, Dialkylamino) seien geradkettige oder verzweigte C₁-C₆-Alkylgruppen genannten, wie Methyl, Ethyl, Propyl, Isopropyl, Butyl, Isobutyl, tert.-Butyl, die isomeren Pentyle und Hexyle. In bevorzugter Weise seien die genannten C₁-C₄-Alkylgruppen genannt, besonders bevorzugt Methyl und Ethyl, ganz besonders bevorzugt Methyl.

Als Aryl in den genannten Substituenten (Aryl, Aryloxy, Arylamino) seien Phenyl oder ein in 2-, 3- oder 4-Stellung gebundenes 5- oder 6-gliedriges Heteroaryl mit 1 oder 2 Heteroatomen aus der Gruppe N-O-S genannt. Beispiele für solches Heteroaryl sind: Furanyl, Pyrrolyl, Thienyl, Pyrazolyl, Imidazolyl, Oxazolyl, Thiazolyl, Pyridyl und andere. In bevorzugter Weise steht Phenyl für Aryl.

Als C₃-C₆-Cycloalkyl sei beispielsweise Cyclopropyl, Cyclobutyl, Cyclopentyl und Cyclohexyl, bevorzugt Cyclopropyl, Cyclopentyl und Cyclohexyl, genannt. Die Cycloalkyl-Substituenten können ihrerseits ein- oder zweimal durch Methyl oder Ethyl substituiert sein.

Als Halogen sei beispielsweise Fluor, Chlor, Brom, Iod, bevorzugt Fluor, Chlor, Brom, besonders bevorzugt Fluor und Chlor, genannt.

Der Ausdruck "X" im Cycloalkylring in den Formeln (I), (IV) und (V) kann die Methylengruppe -CH₂- oder eine zwischen den benachbarten C-Atomen durchgehende Bindung bedeuten. Im ersteren Fall wird hiermit das Cyclohexangerüst, im zweiten Fall das Cyclopentangerüst ausgedrückt. In bevorzugter Weise bedeutet X die Methylengruppe und stellt somit das Cyclohexangerüst dar.

Folgende Cycloalkanone seien als Ausgangsverbindungen im einzelnen beispielhaft aufgeführt:
Cyclopentanon, 2-Methyl-cyclopentanon, 2-Ethyl-cyclopentanon, 2-Phenyl-cyclopentanon, Cyclohexanon, 2-Methyl-cyclohexanon, 4-Methyl-cyclohexanon, 2-Ethyl-cyclohexanon, 4-Ethyl-cyclohexanon, 4-Phenyl-cyclohexanon, 2-Phenyl-cyclohexanon, 4-Cyclohexyl-cyclohexanon, 2-Cyclohexyl-cyclohexanon.

Folgende Aniline als Ausgangsverbindungen seien im einzelnen beispielhaft aufgeführt:
Anilin, 2-Methylanilin, 3-Methylanilin, 4-Methylanilin, 2,4-Dimethyl-anilin, 2-Ethylanilin, 4-Ethylanilin, 3-Methoxyanilin, 3-Ethoxyanilin, 4-Aminoanilin, 4-Methylamino-anilin, 4-Dimethylamino-anilin, 4-Phenylanilin, 2-Phenylanilin, 4-Phenoxyanilin, 2-Phenoxyanilin, 4-Phenylamino-anilin, 2-Phenylamino-anilin, 2-Fluoranilin, 4-Fluoranilin, 2-Chloranilin, 4-Chloranilin.

Das Molverhältnis, in welchem das Cycloalkanon und das Anilin zudosiert werden, beträgt von 2:1 bis 1:2, bevorzugt von 1,5:1 bis 1:1,5, besonders bevorzugt von 1,2:1 bis 1:1,2, ganz besonders bevorzugt von 1,1:1 bis 1:1,1.

Zur azeotropen Entfernung des Reaktionswassers können Azeotropbildner aus der Gruppe der aliphatischen und aromatischen Kohlenwasserstoffe, der aliphatischen und aromatischen Ether sowie der aliphatischen Alkohole eingesetzt werden. In diesen Verbindungen können Wasserstoffatome durch Halogenatome ersetzt sein. Diese Azeotropbildner, ihre Wirkungsweise und ihr Einsatz sind dem Fachmann grundsätzlich bekannt. Als Azeotropbildner seien beispielhaft folgende aufgeführt:
Benzol, Toluol, Xylol, Anisol, Chlorbenzol, Butanol, Hexanol, Methyl-butylether, Diphenylether, p-Chloranisol. Das Gewichtsverhältnis, in dem ein Azeotropbildner und die Summe der Ausgangsstoffe zudosiert werden, beträgt 0,001-1:1, bevorzugt 0,001-0,5:1, besonders bevorzugt 0,001-0,2:1, ganz besonders bevorzugt 0,001-0,1:1. In einer dem Fachmann bekannten Weise kann das abdestillierte Azeotrop außerhalb des kolonnenartigen Reaktors kondensiert und in Reaktionswasser und Azeotropbildner geschieden werden. Der so zurückerhaltende Azeotropbildner kann erneut in die Reaktion des erfindungsgemäßen Verfahren zurückgeführt werden.

Als Azeotropbildner kann auch einer der Ausgangsstoffe, bevorzugt das Cycloalkanon dienen, so daß auf einen systemfremden Azeotropbildner verzichtet werden kann.

Als saure heterogene Katalysatoren kommen saure anorganische oder organische Festkörper infrage. Saure organische Festkörper sind beispielsweise saure Ionenaustauscher, bevorzugt mit makroporöser Struktur, wie sie aus DE-AS 1 113 570, DE-OS 1 595 700 und DE-OS 2 525 295 bekannt sind. Es handelt sich hierbei um sulfonierte Polystyrolharze und Polyacrylsäureharze. Desweiteren sind perfluorierte organische sulfonierte Harze verwendbar. Ebenso können saure Phenol-Formaldehyd- oder Phenol-Melamin-Harze eingesetzt werden.

Als anorganische, sauer wirkende Festkörper seien Oxide von Metallen mit mittlerer und hoher Oxidationstufe und deren Mischoxide genannt. Neben Metalloxiden sind auch die Oxide einiger Nichtmetalle oder Halbmetalle geeignet. Als solche Metalle, Nichtmetalle und Halbmetalle seien beispielsweise genannt:
Bor³⁺, Aluminium³⁺, Seltenerden³⁺, Silicium⁴⁺, Germanium⁴⁺, Zinn⁴⁺, Titan⁴⁺, Zirkonium⁴⁺, Hafnium⁴⁺, Vanadium³⁺, Vanadium⁵⁺, Niob⁵⁺, Tantal⁵⁺, Chrom³⁺, Chrom⁶⁺, Molybdän⁶⁺, Wolfram⁶⁺, Zink²⁺. Besonders bevorzugt hierunter sind saure Oxide und Mischoxide mit einem Gehalt an Aluminiumoxid, besonders bevorzugt saure Aluminiumsilikate, beispielsweise solche mit Schichtstruktur (sogenannte Clays) und saure Aluminosilikate mit definierter Mikroporosität (Zeolithe).

Beispiele für saure anorganische oxidische Festkörper sind:
Böhmit, γ-Al₂O₃, Montmorillonit, Bentonit, Glimmer, H-ZSM 11, H-ZSM 5, H-Mordenit, H-Y, SE-Y, H-Q, H-L, H-Offretit, H-ZSM12, H-ZSM23, H-Ferrierit. Anorganische Oxide und Mischoxide haben für den erfindungsgemäßen Einsatz eine BET-Oberfläche von 40-400 m²/g, bevorzugt 50-350 m²/g, besonders bevorzugt 100-300 m²/g. Speziell bei Zeolithen liegt der Bereich der BET-Oberfläche bei 20-800 m²/g, bevorzugt 30-600 m²/g, besonders bevorzugt 40-400 m²/g. Der pH-Wert einer Aufschlämmung solcher anorganischer Oxide oder Mischoxide von 1 g feingemahlenem Oxid oder Mischoxid in 40 ml 1n wäßriger NaCl-Lösung sollte zwischen 0 und 7 betragen und bevorzugt 1-6, besonders bevorzugt 2-5 sein.

Die Form solcher als saure heterogene Katalysatoren einsetzbaren organischen oder anorganischen Festkörper kann prinzipiell beliebig sein. Um die Belastbarkeit des kolonnenartigen Reaktors zu maximieren, ist jedoch vor allem für einen wenig gehinderten Gasstrom (möglichst geringen Druckverlust in der Kolonne) zu sorgen. Dies wird unter anderem dadurch erreicht, daß man die Form und Größe der Teilchen entsprechend der Form und Größe gängiger Füllkörper für Destillationskolonnen wählt. Hierzu werden die anorganischen oder organischen Festkörper mit geeigneten Bindern, wie sie dem Fachmann bekannt sind, zu entsprechend stückigen Füllkörpern, zu Extrudaten, zu Raschigringen, Pallringen, Sattelkörpern und anderen ausgeformt. Die organischen oder anorganischen Festkörper können jedoch auch als feines Pulver auf inerten Keramikkörpern geeigneter Form aufgebracht und gebunden werden. Die Techniken zur Herstellung solcher Formteile sind dem Fachmann prinzipiell bekannt. Unter den genannten organischen und anorganischen Festkörpern sind wegen der höheren thermischen Beständigkeit die anorganischen Festkörper bevorzugt.

Die Belastung der genannten organischen oder anorganischen Festkörper als saure heterogene Katalysatoren liegt ausgedrückt in g/g.h bei 0,1-10, bevorzugt bei 0,2-6, besonders bevorzugt bei 0,3-5 und ganz besonders bevorzugt bei 0,4-3.

Das erfindungsgemäße Verfahren ist dadurch ausgezeichnet, daß die Kondensation in einem für kontinuierliche Reaktionsführung geeigneten kolonnenartigen Reaktor ausgeführt wird, an dessen Längsachse ein Temperaturprofil angelegt wird. Der kolonnenartige Reaktor wird mit Ausnahme von Zugabe- und Abnahmebereichen für die Reaktionspartner und -produkte mindestens teilweise mit einem sauren heterogenen Katalysator in stückiger, oben beschriebener Form, etwa im Sinne einer Destillationskolonne gefüllt. Für den Fall, daß der kolonnenartige Reaktor nicht vollständig mit dem sauren heterogenen Katalysator gefüllt ist, enthalten die nicht mit Katalysator gefüllten Abschnitte inerte Füllkörper, wie sie für Destillationszwecke dem Fachmann bekannt sind, in einer Formgebung etwa der oben genannten Art oder solche nicht mit Katalysator gefüllten Abschnitte des Reaktors enthalten Kolonnenböden, etwa in Form von Glockenböden. Selbstverständlich sind die Abschnitte des kolonnenartigen Reaktors, die mit Katalysator bzw. mit inerten Füllkörpern gefüllt sind, durch Siebböden geeigneter Maschenweite begrenzt, um ein Ausschwemmen des Katalysators bzw. der Füllkörper zu verhindern. Auch die Füllkörper-Abschnitte des Reaktors können Glockenböden enthalten, die in der beschriebenen Weise mit Sieben versehen sind.

Grundsätzlich kann der kolonnenartige Reaktor in verschiedenen Abschnitten seiner Gesamtlänge, insbesondere in den Abschnitten mit Katalysatorfüllung, aufgeweitet oder verengt sein. Wegen der einfacheren Handhabung bei der Beschickung des Reaktors mit Katalysator bzw. Füllkörpern wird jedoch bevorzugt ein Rohr ohne solche Radiusänderungen oder allenfalls mit einer Aufweiterung als Reaktor benutzt. Der Katalysator befindet sich im Rahmen der mindestens teilweisen Befüllung in einem beliebigen Abschnitt des kolonnenartigen Reaktors unter Berücksichtigung des weiter unten beschriebenen Temperaturprofils. Als ein solcher beliebiger Abschnitt für die Beschickung mit Katalysator sei ein Bereich zwischen dem oberen und dem unteren Achtel, bevorzugt zwischen dem oberen und dem unteren Viertel, besonders bevorzugt zwischen dem oberen und dem unteren Drittel der Gesamtlänge des kolonnenartigen Reaktors genannt.

Dem kolonnenartigen Reaktor wird erfindungsgemäß ein Temperaturprofil angelegt. Dieses Temperaturprofil umfaßt den Temperaturbereich von 10-300°C, bevorzugt 15-250°C, besonders bevorzugt 20-200°C. Zur Einstellung des Temperaturgradienten in den einzelnen Abschnitten des kolonnenartigen Reaktors werden diese Abschnitte mit einer Isolierung bzw. mit einer Thermostatisierung versehen, wie es Stand des chemischen Apparatebaus ist. Die Thermostatisierung kann hierbei je nach Bedarf eine Heizung oder eine Kühlung darstellen.

Die Abschnitte des kolonnenartigen Reaktors, die mit saurem heterogenem Katalysator gefüllt sind, sollten eine Temperatur von höchstens 250°C, bevorzugt höchstens 200°C, besonders bevorzugt höchstens 150°C aufweisen.

Der Temperaturbereich für solche mit Katalysator gefüllten Abschnitte beträgt demnach bevorzugt 25-160°C, besonders bevorzugt 30-150°C, ganz besonders bevorzugt 40-140°C. Die genannte Temperatureinstellung für die mit Katalysator beschickten Abschnitte des Reaktors kann in genannter Weise durch eine Isolierung oder eine vollständige oder teilweise Thermostatisierung, bevorzugt durch eine Isolierung eingehalten werden.

Der Abschnitt des Reaktors oberhalb der Katalysatorfüllung weist eine niedrigere Temperatur als die der Katalysatorfüllung auf, beispielsweise 15-100°C, bevorzugt 10-80°C, besonders bevorzugt 5-70°C weniger als die Katalysatorfüllung. Der Abschnitt des Reaktors unterhalb der Katalysatorfüllung weist eine höhere Temperatur als die der Katalysatorfüllung auf, beispielsweise 20-100°C, bevorzugt 25-90°C, besonders bevorzugt 25-80°C mehr als die Katalysatorfüllung. Die niedrigere Temperatur oberhalb der Katalysatorfüllung kann hierbei zum oberen Reaktorende hin kontinuerlich oder in mehreren Stufen abnehmen. Die Temperatur des Reaktors unterhalb der Katalysatorfüllung kann in entsprechender Weise zum unteren Reaktorende hin kontinuierlich oder in mehreren Abschnitten zunehmen. Je nach gewünschter Einstellung dieser abnehmenden bzw. zunehmenden Temperatur kann dies durch bloße Isolierung oder durch entsprechende Thermostatisierung (Kühlung bzw. Heizung) erfolgen. Auch längs der Katalysatorfüllung kann ein Temperaturgradient angelegt werden mit niedrigeren Temperaturen nach oben und höheren Temperaturen nach unten. In jedem Falle ist der kolonnenartige Reaktor an seinem unteren Ende beheizt und zwar über eine Heizschlange, einen Heizmantel oder eine externe Heizung. Bevorzugt gehört ferner zur Ausrüstung des kolonnenartigen Reaktors ein Sumpfumlauf des als Verfahrensprodukt zu entnehmenden Azomethins. Die Temperatur des Heizmediums für die Heizung des unteren Kolonnenabschnitts mit dem Sumpfumlauf sollte nicht höher als 100°C, bevorzugt nicht hoher als 80°C, besonders bevorzugt nicht höher als 60°C, ganz besonders bevorzugt nicht höher als 40°C über der Siedetemperatur des als Produkt zu entnehmenden Azomethins unter dem jeweiligen in der Kolonne herrschenden Druck liegen.

In bevorzugter Weise wird unterhalb der Katalysatorfüllung eine zusätzliche Thermostatisierung angebracht, die eine Temperatur zwischen der Katalysatorfüllung und dem Reaktorende einzustellen gestattet. Die Heizflüssigkeit für diese Thermostatisierung soll eine Rücklauftemperatur von nicht höher als 60°C, bevorzugt nicht höher als 40°C, besonders bevorzugt nicht hoher als 20°C oberhalb der Siedetemperatur des Kolonneninhalts an dieser Stelle unter dem dort herrschenden Betriebsdruck haben.

Das erfindungsgemäße Verfahren wird in einem Druckbereich von 0,5 mbar bis 3 bar, bevorzugt von 1 mbar bis 1 bar, besonders bevorzugt bei 2 - 250 mbar, ganz besonders bevorzugt bei 3 - 180 mbar, durchgeführt. Für die bevorzugte Durchführung im Unterdruckbereich ist der kolonnenartige Reaktor an seinem oberen Niedertemperaturende mit einer Vakuumpumpe ausgerüstet, die vor oder hinter den Kondensator für das azeotrop zu entnehmende Wasser eingeschaltet werden kann.

Die Eingangsstoffe können einzeln oder als Mischung oberhalb des Katalysators an einem beliebigen Punkt im Niedertemperaturbereich in den Reaktor eingespeist werden. Bevorzugt werden sie als Gemisch unmittelbar oberhalb der Katalysatorfüllung eingespeist. In besonders bevorzugter Weise wird das Gemisch an dem Punkt in den Reaktor eingespeist, an dem das Verhältnis der Einsatzstoffe in der flüssigen Phase im Reaktor gleich ist dem Verhältnis der einzuspeisenden Mischung. Für den Fall, daß einer der Einsatzstoffe infolge seiner größeren Flüchtigkeit in der Katalysatorfüllung unterrepräsentiert ist, kann er jedoch ganz oder auch teilweise unterhalb der Katalysatorfüllung eingespeist und auf diese Weise dem anderen Reaktionspartner ganz oder teilweise im Gegenstrom entgegengeführt werden.

Wird ein separater Azeotropbildner eingesetzt, kann er ebenfalls getrennt von den Ausgangstoffen eingespeist werden; in bevorzugter Weise wird er jedoch mit der Mischung der Ausgangsstoffe oder zumindest gemischt mit einem der Ausgangsstoffe eingespeist.

Das Azomethin als Reaktionsprodukt wird am unteren Reaktorende im Hochtemperaturbereich, beispielsweise aus dem Sumpfumlauf abgeführt. Das Produkt ist normalerweise sehr rein und kann ohne weitere Reinigung weiter verwendet werden. Für den Fall, daß schwerflüchtige Nebenprodukte abgetrennt werden sollen, kann das gewünschte Azomethin in einer grundsätzlich dem Verfahrenstechniker bekannten Weise an einem Punkt oberhalb der Abnahme solcher schwerflüchtiger Nebenprodukte dem kolonnenartigen Reaktor entnommen werden und zwar dort, wo eine maximale Reinheit gewährleistet ist.

In einer bevorzugten Form wird dem im Hochtemperaturbereich entnommenen Azomethin ein ebenfalls im Hochtemperaturbereich, d.h. in jedem Falle unterhalb der Katalysatorfüllung eingespeister Inertgasstrom entgegengeführt. Als Inertgas sei beispielsweise genannt: Luft, Stickstoff, Argon oder Methan, die alle bevorzugt in getrockneter Form eingesetzt werden.

Am oberen Ende des kolonnenartigen Reaktors, also im Niedertemperaturbereich wird Reaktionswasser als Azeotrop entnommen und in einem Scheidegefäß in eine wäßrige und in eine organische Phase des Azeotropbildners getrennt. Die organische Phase wird in bevorzugter Weise dem Reaktor wieder zugeführt, insbesondere, wenn einer der Reaktionspartner gleichzeitig der Azeotropbildner ist; dies kann an einer der oben genannten Stellen erfolgen.

Der Vorteil des erfindungsgemäßen Verfahrens gegenüber dem Stand der Technik besteht in der Möglichkeit des nahezu äquimolaren Einsatzes der Ausgangsstoffe, die sodann vollständig in einem einzigen Durchgang umgewandelt werden können. Hierbei wird unter Vermeidung von zusätzlichen Aufarbeitungsschritten eine quantitative Ausbeute erzielt. Der anzuwendende Reaktor ist durch große Einfachheit und durch optimale Ausnutzung der eingesetzten Energie ausgezeichnet.

Die erfindungsgemäß herstellbaren Azomethine sind Ausgangsstoffe für die Hydrierung zu Cyclohexyl-cycloalkylaminen und Phenyl-cycloalkylaminen und für den Fall, daß das Cycloalkanon ein Cyclohexanon ist, zur Dehydrierung zu gegebenenfalls substituierten Diphenylaminen.

### Beispiele

### Aciditätsbestimmung

Zur Bestimmung der Oberflächenacidität wurde 1 g eines zu Pulver zermahlenen Katalysator-Granulats in 40 ml 1n wäßriger NaCl-Lösung suspendiert. Der pH-Wert der Suspension wurde mit einer Glaselektrodenmeßkette bestimmt und die Säuremenge bei einer Tropfgeschwindigkeit von 0,1 ml/min mit 0,1 N NaOH potentiometrisch titriert.
Die Meßwerte sind in Tabelle 1 wiedergegeben.

Die Aufmahlung der Granulat-Proben erfolgte in einem Mikro-Dismembrator (Fa. Braun, Melsungen AG). In den Unterteil einer Mahlvestole wurden 5 ml des Granulats und eine Wolframcarbid-Kugel mit einem Durchmesser von 9 mm gegeben. Die Vestole wurde mit dem Oberteil verschlossen und in den Schüttelbehälter aus PTFE gegeben. Der Schüttelbehälter wurde in die Halterung des Dismembrators eingespannt und die Schwingamplitude auf ca. 10 mm Ausschlag eingestellt. Nach 6 bis 8 Min. war die Mahlung beendet. Das feinteilige Pulver konnte dann für die obige Aciditätsbestimmung eingesetzt werden.

### Beispiel 1

Eine 80 cm lange verspiegelte Vakuumkolonne mit einem Innendurchmesser von 2,5 cm wurde auf eine Höhe von 15 cm mit Raschigringen aus Glas befüllt, auf 65 cm mit 200 g Zeolith H-ZSM 11-Granulat mit den Abmessungen 2 x 6 mm, gebunden mit 15 % SiO₂, aufgefüllt und die Packung mit 15 cm Raschigringen ergänzt. Die Kolonne wurde am unteren Ende mit einem 30 cm langen thermostatisierbaren, mit Raschigringen gefüllten Verdampferrohr mit einem Innendurchmesser von 2,5 cm versehen. Der Kolonnenkopf bestand aus einer Destillationsbrücke mit Thermometer und Dosiertropftrichter. Sowohl Verdampfer als auch Brücke wurden je mit einer Vakuumentnahmeeinheit zum Ausschleusen von Flüssigkeiten versehen. Die untere Einheit wurde an ein Rotameter mit Membrandosierventil angeschlossen. Die Destillationsbrücke wurde mit einer Wasserstrahlpumpe mit elektronisch geregeltem Endvakuum verbunden und der Druck auf 60 mbar eingestellt.
Es wurde ein Stickstoffgegenstrom von 14 l N₂/min bei 60 mbar eingestellt.

Eine Mischung von Cyclohexanon und Anilin im Molverhältnis 1,2:1,0 tropfte mit einer Geschwindigkeit von 120 g/h auf die oberste Packung von Raschigringen. Der Verdampfer wurde auf 180°C thermostatisiert. Nach einer kurzen Anlaufphase stellten sich stationäre Bedingungen ein. Während am Verdampferausgang 98,6 g/h 100 %iges Anil, verunreinigt mit Spuren von weniger als 0,1 % an höhersiedenden Komponenten, austraten, kondensierte am Kolonnenkopf bei einer Temperatur von 45 bis 48°C eine Mischung aus 10,2 g/h Wasser und 11,2 g/h Cyclohexanon.
Die Apparatur wurde ohne Desaktivierung des Katalysators 1000 Std. betrieben.

### Beispiel 2

Eine 90 cm-Kolonne mit einem 15 cm langen thermostatisierbaren Bereich 30 cm vom unteren Ende entfernt wurde mit der in Beispiel 1 beschriebenen Peripherie umgeben. Die Apparatur wurde auf 45 cm, d.h. bis zum Ende des Flüssigkeitsmantels mit Raschigringen gefüllt. Danach wurde eine 20 cm dicke Schicht aus 45 g KAO, einem protonengetauschten Montmorillonit der Fa. Südchemie, mit Teilchengrößen von 3-4 mm, hinzugegeben und die Säule mit Raschigringen aufgefüllt. Bei einem Stickstoffgegenstrom von 2 bis 3 l/h bei 60 mbar wurden 45 g einer Mischung aus 1,2 Molen Cyclohexanon und 1 Mol Anilin pro Stunde zudosiert. Die Verdampfertemperatur war auf 200°C eingestellt, der Flüssigkeitsmantel auf 105°C thermostatisiert. Am Verdampferausgang fielen 37 g/h Anil an, während am Kolonnenkopf das Azeotrop, bestehend aus 3,9 g Wasser und 4,2 g Cyclohexanon bei einer Temperatur von 45-48°C kondensierte. Die Apparatur wurde 100 Stunden ohne Aktivitätsverlust betrieben. Der Versuch wurde ohne Anzeichen auf einen beginnenden Aktivitätsverlust beendet.

### Beispiel 3

Eine 135 cm-Kolonne (Innendurchmesser 2,5 cm) mit einem 20 cm langen thermostatisierbaren Bereich 35 cm vom unteren Ende entfernt und mit einem 10 cm langen thermostatisierbaren Bereich 95 cm vom unteren Ende entfernt wurde mit der im Beispiel 1 beschriebenen Peripherie versehen. In die 40 cm lange isolierte Zone zwischen den thermostatisierbaren Bereichen wurde 110 g KAO (2-3 mm) gegeben, der Rest der Säule erhielt eine Füllung aus Raschigringen. Bei einem Stickstoffgegenstrom von 0,1 bis 0,2 1/h bei 60 mbar wurden 275 g einer Mischung aus 1,18 Mol Cyclohexanon und 1 Mol Anilin pro Stunde in den oberen thermostatisierbaren Bereich dosiert. Die Verdampfertemperatur war auf 210°C, die des unteren thermostatisierten Bereichs auf 115°C, die des oberen thermostatisierten Bereichs auf 35°C eingestellt. Am Verdampferausgang fielen 228 g/h Anil an, während am Kolonnenkopf das Azeotrop, bestehend aus 23,7 g/h Wasser und 23,3 g/h Cyclohexanon bei 39-40°C kondensierte. Die Apparatur wurde 800 Stunden ohne Aktivitätsverlust betrieben und der Versuch ohne Anzeichen einer beginnenden Desaktivierung beendet. Die folgende Tabelle 1 zeigt die mit dem Katalysator KAO erzielten Ergebnisse und Ergebnisse, die mit weiteren Katalysatoren erzielt wurden.

**Tabelle 1**

| Katalysatoren, die in Beispiel 3 eingesetzt wurden, die gemessenen pH-Werte, sowie die mit ihnen erzielten Umsätze und Selektivitäten. | | | | |
|---|---|---|---|---|
| Katalysator | Bemerkungen | pH | Ums. % | Selek. % |
| Al₂O₃ | Pural SCF (Fa. Condea) | 6,8 | 100 | 97 |
| | SPH 512 (Fa. Rh.-Poulenc) | 9,1 | 20* | 100 |
| | Disperal spez 10/1 (Fa. Condea) | 6,9 | 100 | 99 |
| | Al₂O₃ aktiv (Fa. Johnson Matthey) | 9,4 | 10* | 100 |
| Schichtsilikate | KAO (Südchemie) | 4,1 | 100 | 100 |
| | KA1 (Südchemie) | 3,4 | 100 | 100 |
| | KA2 (Südchemie) | 4,2 | 100 | 100 |
| | KA3 (Südchemie) | 5,0 | 100 | 100 |
| | K306 (Südchemie) | 3,5 | 100 | 100 |
| | KP-10 (Südchemie) | 2,4 | 100 | 100 |
| | KS (Südchemie) | 2,8 | 100 | 98 |
| | KSF/o (Südchemie) | 1,9 | 100 | 100 |
| | KSF (Südchemie) | 2,3 | 100 | 100 |
| Zeolithe (Module), alle gebunden mit 15 % SiO₂ | H-ZSM 11 (110) | 4,0 | 100 | 100 |
| | " ( 54) | 2,4 | 100 | 100 |
| | H-ZSM 5 (326) | 2,9 | 100 | 100 |
| | " (180) | 2,8 | 100 | 100 |
| | H-Mordenit (12,5) | 2,9 | 100 | 100 |
| | H-Y (25,0) | 3,9 | 100 | 100 |
| | SE-Y (12,0) | 4,4 | 100 | 100 |

| | | | | |
|---|---|---|---|---|
| * Es stellten sich nicht die in Beispiel 3 aufgezeigten Bedingungen ein, vielmehr destillierte der größte Teil der zudosierten Eduktmischung über den Kolonnenkopf ab. | | | | |

## Patentansprüche

1. Verfahren zur Herstellung von Azomethinen der Formel 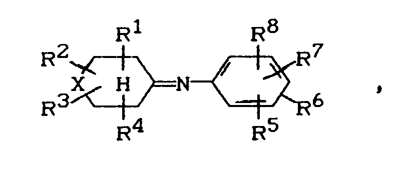 in der
R¹, R², R³, R⁴, R⁵, R⁶, R⁷ und R⁸ unabhängig voneinander Wasserstoff, geradkettiges oder verzweigtes C₁-C₆-Alkyl, C₃-C₆-Cycloalkyl, Benzyl oder Aryl bedeuten und
R⁵, R⁶, R⁷ und R⁸ unabhängig voneinander zusätzlich Halogen, geradkettiges oder verzweigtes C₁-C₆-Alkoxy, Hydroxy, Amino, C₁-C₆-Alkylamino, Di-C₁-C₆-alkyl-amino, Aryloxy oder Arylamino bedeuten,
wobei Aryl für Phenyl oder für in 2-, 3- oder 4-Stellung gebundenes 5- oder 6-gliedriges Heteroaryl mit 1 oder 2 Heteroatomen aus der Gruppe N, O und S steht, und
X -CH₂- oder eine zwischen den benachbarten C-Atomen durchgehende Bindung bedeutet,
durch Kondensation von Cycloalkanonen der Formel 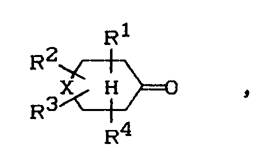 mit Anilinen der Formel 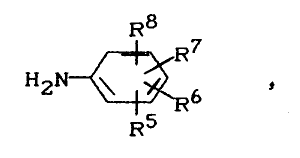 in denen R¹ bis R⁸ und X die obige Bedeutung haben,
in Gegenwart von sauren heterogenen Katalysatoren unter azeotroper Entfernung des Reaktionswassers, dadurch gekennzeichnet, daß die Kondensation in kontinuierlicher Reaktion in einem kolonnenartigen Reaktor mit einem angelegten Temperaturprofil im Bereich von 10 - 300°C durchgeführt wird, wobei die mit Katalysator gefüllten Reaktorabschnitte eine Temperatur von höchstens 250°C aufweisen und die Ausgangsstoffe im Niedertemperaturbereich zugeführt werden und von den Reaktionsprodukten das azeotrop zu entfernende Reaktionswasser ebenfalls im Niedertemperturbereich und das entstehende Azomethin im Hochtemperaturbereich abgeführt werden und wobei bei einem Molverhältnis von Cyclohexanon:Anilin = 2:1 bis 1:2 und in einem Druckbereich von 0,5 mbar bis 3 bar gearbeitet wird.

2. Verfahren nach Anspruch 1, dadurch gekennzeichnet, daß Cycloalkanone der Formel 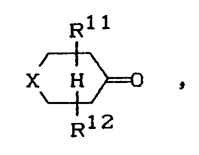 eingesetzt werden, worin
R¹¹ und R¹² unabhängig voneinander Wasserstoff, geradkettiges oder verzweigtes C₁-C₄-Alkyl, Cyclopropyl, Cyclopentyl, Cyclohexyl, Benzyl oder Phenyl bedeuten und
X -CH₂- oder eine zwischen den benachbarten C-Atomen durchgehende Bindung bedeutet
und bevorzugt Cycloalkanone der Formel 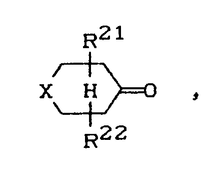 eingesetzt werden, worin
R²¹ und R²² unabhängig voneinander Wasserstoff, Methyl oder Ethyl bedeuten und
X die obige Bedeutung hat.

3. Verfahren nach Anspruch 1, dadurch gekennzeichnet, daß die Cycloalkanone Cyclohexanone sind.

4. Verfahren nach Anspruch 1, dadurch gekennzeichnet, daß Aniline der Formel 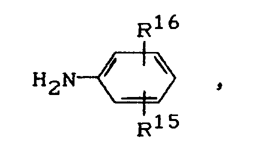 eingesetzt werden, in der
R¹⁵ und R¹⁶ unabhängig voneinander Wasserstoff, geradkettiges oder verzweigtes C₁-C₄-Alkyl, Phenyl, Fluor, Chlor, Brom, geradkettiges oder verzweigtes C₁-C₄-Alkoxy, Hydroxy, Amino, C₁-C₄-Alkylamino, Di-C₁-C₄-alkyl-amino, Phenoxy oder Phenylamino bedeuten
und bevorzugt Aniline der Formel 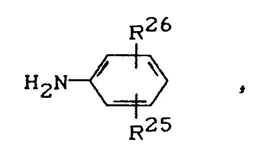 eingesetzt werden, in der
R²⁵ und R²⁶ unabhängig voneinander Wasserstoff, Methyl, Ethyl, Fluor, Chlor, Methoxy, Ethoxy, Methylamino, Ethylamino, Dimethylamino oder Diethylamino bedeuten
und besonders bevorzugt Aniline der Formel 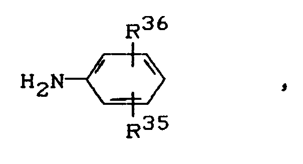 eingesetzt werden, in der
R³⁵ und R³⁶ unabhängig voneinander Wasserstoff, Fluor, Chlor, Methyl, Ethyl, Methoxy, Methylamino oder Dimethylamino bedeuten.

5. Verfahren nach Anspruch 1, dadurch gekennzeichnet, daß das Cycloalkanon und das Anilin im molaren Verhältnis von 1,5:1-1:1,5, bevorzugt 1,2:1-1:1,2, besonders bevorzugt 1,1:1-1:1,1 eingesetzt werden.

6. Verfahren nach Anspruch 1, dadurch gekennzeichnet, daß der kolonnenartige Reaktor mit Ausnahme von Zugabe- und Abnahmebereichen für die Reaktionspartner und -produkte mindestens teilweise mit saurem heterogenen Katalysator in stückiger Form gefüllt ist.

7. Verfahren nach Anspruch 6, dadurch gekennzeichnet, daß die nicht mit Katalysator gefüllten Abschnitte des kolonnenartigen Reaktors mit inerten Füllkörpern in stückiger Form gefüllt sind oder mit Destillationsböden, bevorzugt mit Glockenböden, ausgerüstet sind.

8. Verfahren nach Anspruch 1, dadurch gekennzeichnet, daß das angelegte Temperaturprofil im Bereich von 15-250°C, bevorzugt 20-200°C liegt, wobei die mit Katalysator gefüllten Reaktorabschnitte eine Temperatur von höchstens 200°C, bevorzugt höchstens 150°C aufweisen.

9. Verfahren nach Anspruch 1, dadurch gekennzeichnet, daß dem im Hochtemperaturbereich entnommenen Azomethin ein ebenfalls im Hochtemperaturbereich eingespeister Inertgastrom entgegengeführt wird.

10. Verfahren nach Anspruch 1, dadurch gekennzeichnet, daß bei einem Druck von 1 mbar-1 bar, bevorzugt von 2-250 mbar, besonders bevorzugt von 3-180 mbar gearbeitet wird.

## Claims

1. Process for the preparation of azomethines of the formula 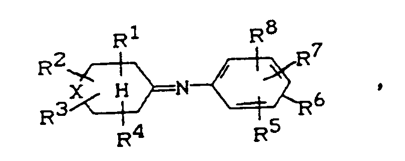 in which
R¹, R², R³, R⁴, R⁵, R⁶, R⁷ and R⁸ independently of one another denote hydrogen, linear or branched C₁-C₆-alkyl, C₃-C₆-cycloalkyl, benzyl or aryl, and
R⁵, R⁶, R⁷ and R⁸ independently of one another addi tionally denote halogen, linear or branched C₁-C₆-alkoxy, hydroxyl, amino, C₁-C₆-alkylamino, di-C₁-C₆-alkylamino, aryloxy or arylamino,
aryl representing phenyl or 5-membered or 6-membered heteroaryl attached in the 2-, 3- or 4-position and having 1 or 2 hetero atoms belonging to the group N, O and S, and
X denotes -CH₂- or a direct bond between the adjacent C atoms,
by the condensation of cycloalkanones of the formula 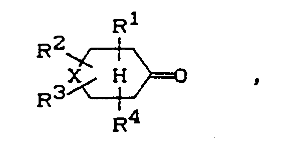 with anilines of the formula 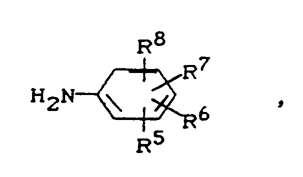 in which R¹ to R⁸ and X have the above meaning,
in the presence of acid heterogeneous catalysts with azeotropic removal of the water of reaction, characterized in that the condensation reaction is carried out in a continuous reaction in a column-like reactor having an applied temperature profile in the range 10-300°C, the sections of the reactor which are packed with catalyst having a temperature not higher than 250°C and the starting materials being fed in in the low-temperature zone and, of the reaction products, the water of reaction to be removed as an azeotrope likewise being removed in the low-temperature zone and the azomethine formed being removed in the high-temperature zone, and a molar ratio of cyclohexanone: aniline of 2:1 to 1:2 and a pressure range from 0.5 mbar to 3 bar being employed.

2. Process according to Claim 1, characterized in that cycloalkanones of the formula 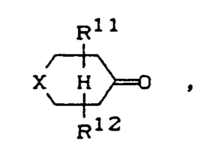 wherein
R¹¹ and R¹² independently of one another denote hydrogen, linear or branched C₁-C₄-alkyl, cyclopropyl, cyclopentyl, cyclohexyl, benzyl or phenyl and
X denotes -CH₂- or a direct bond between the adjacent C atoms,
are employed and, preferably, cycloalkanones of the formula 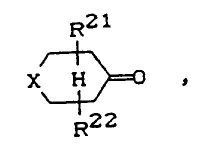 wherein
R²¹ and R²² independently of one another denote hydrogen, methyl or ethyl and
X has the above meaning,
are employed.

3. Process according to Claim 1, characterized in that the cycloalkanones are cyclohexanones.

4. Process according to Claim 1, characterized in that anilines of the formula 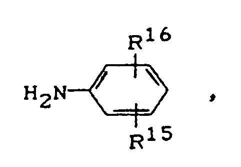 in which
R¹⁵ and R¹⁶ independently of one another denote hydrogen, linear or branched C₁-C₄-alkyl, phenyl, fluorine, chlorine, bromine, linear o r branched C₁-C₄-alkoxy, hydroxyl, amino, C₁-C₄-alkylamino, di-C₁-C₄-alkylamino, phenoxy or phenylamino,
are employed and, preferably, anilines of the formula 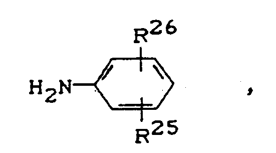 in which
R²⁵ and R²⁶ independently of one another denote hydrogen, methyl, ethyl, fluorine, chlorine, methoxy, ethoxy, methylamino, ethylamino, dim ethylamino or diethylamino,
are employed and, particularly preferably, anilines of the formula 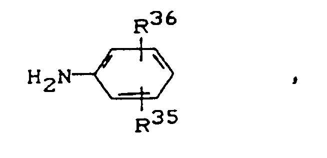 in which
R³⁵ and R³⁶ independently of one another denote hydrogen, fluorine, chlorine, methyl, ethyl, methoxy, methylamino or dimethylamino,
are employed.

5. Process according to Claim 1, characterized in that the cycloalkanone and the aniline are employed in a molar ratio of 1.5:1-1:1.5, preferably 1.2:1-1:1.2 and particularly preferably 1.1:1-1:1.1.

6. Process according to Claim 1, characterized in that the column-like reactor is packed, with the exception of addition and removal zones for the reactants and reaction products, at least partially with an acid heterogeneous catalyst in a pelletized form.

7. Process according to Claim 6, characterized in that the sections of the column-like reactor which are not packed with catalyst are packed with inert packing in a pelletized form or are equipped with distillation plates, preferably bubble cap plates.

8. Process according to Claim 1, characterized in that the temperature profile applied is within the range from 15 to 250°C, preferably 20-200°C, the sections of the reactor packed with catalyst having a temperature not higher than 200°C and preferably not higher than 150°C.

9. Process according to Claim 1, characterized in that a stream of inert gas is fed in in the high-temperature zone countercurrent to the azomethine removed in the high-temperature zone.

10. Process according to Claim 1, characterized in that the reaction is carried out under a pressure of 1 mbar - 1 bar, preferably 2 - 250 mbar and particularly preferably 3 - 180 mbar.

## Revendications

1. Procédé de préparation d'azométhines de formule : 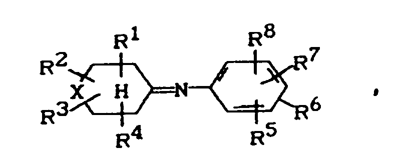 dans laquelle
R¹, R², R³, R⁴, R⁵, R⁶, R⁷ et R⁸ représentent chacun, indépendamment l'un de l'autre, un atome d'hydrogène, un groupe alkyle en C₁ à C₆, linéaire ou ramifié, un groupe cycloalkyle en C₃ à C₆, benzyle ou aryle, et
R⁵, R⁶, R⁷ et R⁸ représentent chacun, indépendamment l'un de l'autre, également un atome d'halogène, un groupe alcoxy en C₁ à C₆, linéaire ou ramifié, un groupe hydroxy, amino, alkylamino en C₁ à C₆, dialkyl(en C₁ à C₆)-amino, aryloxy ou arylamino,
le terme aryle désignant un groupe phényle ou un groupe hétéroaryle pentagonal ou hexagonal, fixé en position 2, 3 ou 4 et comportant 1 ou 2 hétéroatomes choisis parmi N, O et S, et
X représente -CH₂- ou une liaison disposée entre les atomes de carbone voisins,
par condensation de cycloalcanones de formule : 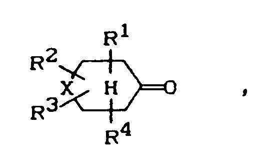 avec des anilines de formule : 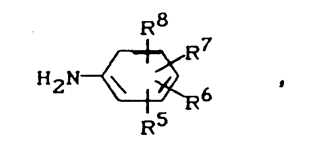 dans lesquelles R¹ à R⁸ et X ont le sens précité,
en présence de catalyseurs hétérogènes acides avec élimination, sous forme d'un azéotrope, de l'eau de réaction, procédé caractérisé en ce qu'on conduit la condensation dans une réaction continue dans un réacteur en forme de colonne à laquelle on applique un profil de température allant de 10 à 300°C, les tronçons de réacteur garnis de catalyseur présentant une température dont la valeur maximale est de 250°C, et les matières de départ étant introduites dans la zone à basse température et l'eau de réaction, à enlever sous forme de l'azéotrope des produits de réaction, étant également éloignée de la zone à basse température et l'azométhine résultante étant prélevée dans la zone à température élevée, et le travail étant effectué avec un rapport molaire entre la cyclohexanone et l'aniline valant de 2 : 1 à 1 : 2 et le travail étant réalisé dans un domaine de la pression allant de 0,5 mbar à 3 bars.

2. Procédé selon la revendication 1, caractérisé en ce qu'on utilise des cyclohexanones de formule : 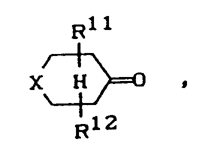 dans laquelle
R¹¹et R¹² représentent chacun, indépendamment l'un de l'autre, un atome d'hydrogène, un groupe alkyle en C₁ à C₄, linéaire ou ramifié, un groupe cyclopropyle, cyclopentyle, cyclohexyle, benzyle ou phényle, et
X représente -CH₂- ou une liaison existant entre deux atomes de carbone voisins,
et l'on utilise de préférence des cyclohexanones de formule : 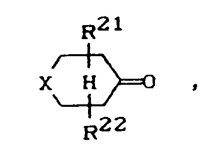 dans laquelle
R²¹ et R²² représentent chacun, indépendamment l'un de l'autre, un atome d'hydrogène, un groupe méthyle ou éthyle, et
X a le sens ci-dessus.

3. Procédé de fabrication 1, caractérisé en ce que les cycloalcanones sont les cyclohexanones.

4. Procédé de fabrication 1, caractérisé en ce qu'on utilise des anilines de formule : 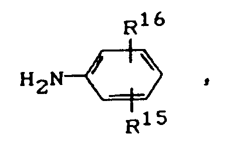 dans laquelle
R¹⁵ et R¹⁶ représentent chacun, indépendamment l'un de l'autre, un atome d'hydrogène, un groupe alkyle en C₁ à C₄, linéaire ou ramifié, un groupe phényle, un atome de fluor, de chlore, de brome, un groupe alcoxy en C₁ à C₄, linéaire ou ramifié, un groupe hydroxy, amino, alkylamino en C₁ à C₄, dialkyle (en C₁ à C₄)-amino, phénoxy ou phénylamino,
et l'on utilise de préférence des anilines de formule : 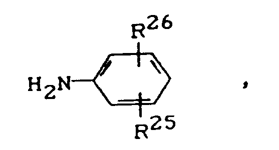 dans laquelle
R²⁵ et R²⁶ représentent chacun, indépendamment l'un de l'autre, un atome d'hydrogène, un groupe méthyle, éthyle, un atome de fluor, de chlore, un groupe méthoxy, éthoxy, méthylamino, éthylamino, diméthylamino et diéthylamino,
et l'on utilise de façon particulièrement préférée des anilines de formule : 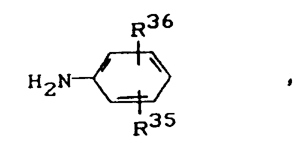 dans laquelle
R³⁵ et R³⁶ représentent chacun, indépendamment l'un de l'autre, un atome d'hydrogène, de fluor, de chlore, un groupe méthyle, éthyle, méthoxy, méthylamino ou diméthylamino.

5. Procédé selon la revendication 1, caractérisé en ce qu'on utilise la cycloalcanone et l'aniline selon un rapport molaire de 1,5 : 1 à 1 : 1,5, de préférence 1,2 : 1 à 1 : 1,2, de façon particulièrement préférée de 1,1 : 1 à 1 : 1,1.

6. Procédé selon la revendication 1, caractérisé en ce que le réacteur en forme de colonne est, à l'exclusion des zones d'introduction et de prélèvement des corps participant à la réaction des produits de la réaction, garni au moins partiellement par du catalyseur hétérogène acide sous forme de morceaux ou pièces.

7. Procédé selon la revendication 6, caractérisé en ce que les tronçons, non garnis de catalyseur, du réacteur en forme de colonne sont garnis par des corps inertes de garnissage sous forme de morceaux, ou bien ils sont équipes de plateaux de distillation, comportant de préférence des plateaux à cloche.

8. Procédé selon la revendication 1, caractérisé en ce que le profil de température appliqué se situe entre 15 et 250°C, de préférence entre 20 et 200°C, les tronçons du réacteur garni de catalyseur présentant une température dont le maximum est de 200°C et de préférence dont le maximum est de 150°C.

9. Procédé selon la revendication 1, caractérisé en ce qu'un courant de gaz inerte, également introduit dans la zone à température élevée, circule à contrecourant de l'azométhine prélevée dans la zone à température élevée.

10. Procédé selon la revendication 1, caractérisé en ce qu'on travaille sous une pression de 1 mbar à 1 bar, de préférence une pression de 2 à 250 mbars et de façon particulièrement préférée une pression de 3 à 180 mbars.
